# EUROPEAN PATENT APPLICATION

(11) **EP 4 728 991 A1**
(43) Date of publication of application: **22.04.2026**
(21) Application number: 24207127.2
(22) Date of filing: 17.10.2024
(51) Int. Cl.: A61B 8/06, A61B 8/08, A61B 8/00, G06T 7/00

(54) **MICROVESSEL SIZE QUANTIFICATION FOR PARAMETRIC ASSESSMENT USING CONTRAST ENHANCED ULTRASOUND**

(71) Applicant: Koninklijke Philips N.V., 5656 AG Eindhoven (NL)
(72) Inventor: HYMAN, Gabriel, Eindhoven (NL); APOSTOLAKIS, Iason Zacharias, Eindhoven (NL); LOUPAS, Thanasis, 5656AG Eindhoven (NL); HERBST, Elizabeth, Eindhoven (NL)
(74) Representative: Philips Intellectual Property & Standards

(57) **Abstract**

An ultrasound imaging system acquires, stores, and displays a sequence of ultrasound images of microvasculature acquired during a cycle of contrast agent wash-in and wash-out. The sequence of images is recalled from memory and a region of interest containing microvessels in one of the images is selected using a user interface. The sizes of the microvessels in the region of interest are measured. A slider control on the user interface is manipulated to select a range of microvessel sizes in the region of interest for assessment.

## Description

This invention relates to medical diagnostic ultrasound systems and, in particular, to ultrasound imaging systems which perform contrast-enhanced ultrasound imaging (CEUS).

Nonlinear contrast agents have been available for many years for enhancing blood flow and tissue perfusion in ultrasound imaging. Contrast agents are solutions of tiny microbubbles which act as strongly nonlinear echogenic reflectors in the bloodstream. Importantly, the scattering properties of microbubbles, and their nonlinear oscillatory behavior, cause their echo returns to exhibit a broad spectrum of energy. These echo signals can be processed to separate out a selected band or bands of frequencies which are used for imaging. Commonly, the second harmonic band of frequencies is used for contrast imaging. The relatively high intensity of the harmonic echo signals returned from microbubble contrast agents enables various parameters of contrast-enhanced blood flow to be measured and compared. Among these parameters are the time-intensity curve of the wash-in and wash-out of contrast agent as a bolus of contrast agent arrives at and flows through a region of interest in the body, such as one containing a cancerous lesion. The time-intensity curve leads to the estimation of several diagnostically useful parameters, including the intensity at the peak of the time-intensity curve and the area under the time intensity curve, which are measures of contrast agent perfusion characteristics within a specific region of interest.

Tumors generally develop a network of microvessels, small blood vessels that nourish a tumor with a supply of blood and promote its growth. A considerable number of studies suggest that tumor growth and metastasis are dependent on the formation of these new blood vessels inside and around a tumor, that is, angiogenesis. It is therefore clinically useful to quantify microvessel density (MVD), the density of the tumor's tiny microvessels, to assess intratumoral angiogenesis and provide an estimation of patient treatment planning and monitoring. The most common technique for calculating MVD involves taking a biopsy of the lesion and histologically identifying and counting the microvessels present in a given region of interest. This method, while clinically useful and accepted, is by nature invasive and inefficient. Recent studies have demonstrated the ability to correlate the aforementioned parameters of contrast-enhanced ultrasound, peak intensity and area under a time-intensity curve, with MVD. Furthermore, there is also interest in using CEUS to quantify microvascular density for the characterization of plaque neo-vascularization. However, while these CEUS-based methods facilitate the non-invasive assessment of tumor angiogenesis and prognosis, the contrast signals produced by the larger feeding vessels of the lesion or other large surrounding vessels can potentially inflate the resulting quantitative indicators of MVD. Accordingly it is desirable to be able to reduce or eliminate the adverse effects of larger vessel flow on CEUS-based assessment of microvascular density.

In accordance with the principles of the present invention, the estimation of parameters indicative of tumor microvascular angiogenesis using contrast-enhanced ultrasound imaging is improved by decreasing or eliminating the effects of larger vessels on such estimation. An ultrasound imaging system is used to acquire a sequence of contrast-enhanced images of a region of interest as contrast agent perfuses the vasculature of the region. Microvessels within the region of interest are segmented and their sizes estimated. A user interface of the ultrasound system is then used to delineate blood vessels to be included in an evaluation by selecting a range of vascular sizes to be included in and/or excluded from a parametric assessment. The desired parametric estimation is then performed using only the contrast perfusion of microvessels of a desired range of sizes.

In a preferred implementation, an ultrasound system of the present invention comprises an ultrasound probe adapted to acquire ultrasonic echo signals from an image field experiencing a flow of a microbubble contrast agent; a beamformer coupled to the ultrasound probe and adapted to produce beamformed echo signals; a contrast image processor coupled to the beamformer, and adapted to produce images of microvessels perfused with the microbubble contrast agent; an image display coupled to the contrast image processor and adapted to display the images of the microvessels perfused with the microbubble contrast agent; a Cineloop memory, coupled to the contrast image processor, and adapted to store a sequence of the images of anatomy perfused with the microbubble contrast agent acquired during wash-in of the microbubble contrast agent; and a microvessel measurement & selection processor, coupled to the Cineloop memory, and adapted to enable selection of a group of microvessels for measurement and to measure sizes of the microvessels, wherein the microvessel measurement & selection processor is further adapted to enable selection of a range of microvessel sizes for assessment.

In the drawings:
Fig. 1 illustrates in block diagram form an ultrasound system constructed in accordance with the principles of the present invention.
Fig. 2 illustrates a representation of an image of microvessels acquired by an ultrasound system in accordance with the present invention.
Fig. 3 illustrates the image of microvessels of Fig. 2 after undergoing skeletonization by point spread function (PSF) thinning.
Fig. 4 illustrates a technique for measuring the diameter of a microvessel of Fig. 2.
Figs. 5a and 5b illustrate a user interface for selecting a region of interest of microvessels.
Figs. 6a and 6b illustrate the segmentation by size of the microvessels selected in Figs. 5a and 5b.
Figs. 7a and 7b illustrate the selection of a reduced range of microvessel sizes.
Figs. 8a and 8b illustrate the selection of an even narrower range of microvessel sizes.
Fig. 9 illustrates a user interface of an ultrasound system which delineates microvessels of a desired range of sizes in a region of interest in accordance with the present invention.
Fig. 10 illustrates a time-intensity curve of contrast agent wash-in, wash-out which is suitable for assessment of microvessel flow parameters in an implementation of the present invention.
Fig. 11 illustrates a flow chart of an exemplary method enabled by the present invention.

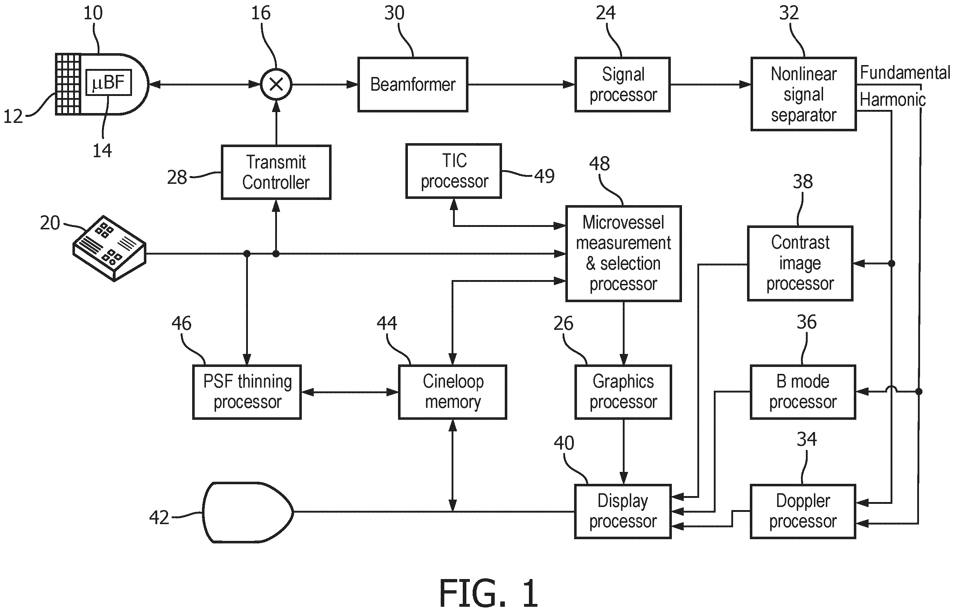

Referring first to Fig. 1, an ultrasound system constructed in accordance with the principles of the present invention is shown in block diagram form. An ultrasound probe 10 includes an array 12 of ultrasonic transducer elements that transmits ultrasonic pulses and receives ultrasonic echo signals. The transducer array 12 may be a one- or two-dimensional array of transducer elements capable of scanning in two or three dimensions, for instance, in both elevation (in 3D) and azimuth. The transducer array 12 in the illustration is a two-dimensional matrix array coupled to a microbeamformer 14 in the probe which controls transmission and reception of signals by the array elements. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) The microbeamformer is coupled by the probe cable to a transmit/receive (T/R) switch 16 which switches between transmission and reception and protects the main beamformer 30 from high energy transmit signals. The transmission of ultrasonic beams from the transducer array 12 under control of the microbeamformer 14 is directed by a transmit controller 28 coupled to the T/R switch, which receives input from the user's operation of the user interface or control panel 20. Among the transmit characteristics controlled by the transmit controller are the frequency, steering, number, spacing, amplitude, phase, and polarity of transmit beams. Beams formed in the direction of pulse transmission may be steered straight ahead from the transducer array, or at different angles for a wider sector field of view or better Doppler reception.

The echoes received by a contiguous group of transducer elements are beamformed by appropriately delaying them and then combining them. The partially beamformed signals produced by the microbeamformer 14 from each patch of elements are coupled to the main beamformer 30 where partially beamformed signals from individual patches of transducer elements are combined into a fully beamformed coherent echo signal. For example, the main beamformer 30 may have 128 channels, each of which receives a partially beamformed signal from a patch of 12 transducer elements. In this way the signals received by over 1500 transducer elements of a two-dimensional array transducer can contribute efficiently to a single beamformed signal.

The coherent echo signals undergo signal processing by a signal processor 24, which includes filtering by a digital filter and noise reduction as by spatial or frequency compounding. The signal processor can also shift the frequency band to a lower or baseband frequency range. The digital filter of the signal processor 24 can be a filter of the type disclosed in U.S. Patent No. 5,833,613 (Averkiou et al.), for example. The echo signals are demodulated by quadrature demodulation into quadrature (I and Q) components, which provide signal phase information for Doppler processing.

The beamformed and processed coherent echo signals are coupled to a nonlinear signal separator 32. The nonlinear signal separator can separate higher harmonic echo signals with a high pass filter, but preferably it separates harmonic frequencies of echoes returned from contrast agent microbubbles by the pulse inversion technique, in which echo signals resulting from the transmission of multiple, differently phased (inverted) pulses to an image location are additively combined to cancel fundamental signal components and enhance harmonic components, thus producing echo signals in a second harmonic band 2fₒ. The harmonic signals can alternatively be separated by amplitude-modulated pulse inversion as described in US Pat. 5,577,505 (Brock-Fisher et al.) The same echo signals are subtractively combined to produce echo signals in a fundamental frequency band fₒ. A preferred phase (polarity) pulse inversion technique is described in U.S. patent 6,186,950 (Averkiou et al.) and in U.S. patent 5,706,819 (Hwang et al.) for instance.

Harmonic echo signals from a contrast agent, such as microbubbles, are coupled to a contrast image processor 38. Contrast agents are often used to more clearly delineate blood vessels, or to perform perfusion studies of the microvasculature of tissue as described in US Pat. 6,692,438 (Skyba et al.) for example, and in an implementation of the present invention. The contrast image processor produces an anatomical contrast image by amplitude (or envelope) detection of the harmonic frequency echoes from each point in the image field. One way to do this when the echoes are quadrature demodulated is to calculate the signal amplitude at each pixel location in the form of (I²+Q²)^{½}. These contrast intensity signals are mapped to the desired display format by scan conversion which converts samples from R-0 coordinates (used in radial scanning) to Cartesian (x,y) coordinates for display of a spatially defined image.

The fundamental frequency echo signals are coupled to a B mode processor 36 which produces a standard B mode tissue image. The B mode processor performs in the same manner as the contrast image processor, but operates on fundamental frequency echoes. The echo signals are amplitude (envelope) detected and scan converted to produce a spatially delineated image of tissue in the image field. Both the fundamental and harmonic echo signals are coupled to a Doppler processor 34. The Doppler processor stores ensembles of echo signals from discrete points in an image field which are then used to estimate the Doppler shift at points in the image, typically by means of a fast Fourier transform (FFT) processor or a lag-one autocorrelation technique. The rate at which the ensembles are acquired determines the velocity range of motion that the system can accurately measure and depict in an image. The Doppler shift is proportional to motion at points in the image field, *e.g.,* blood flow and tissue motion. For a color Doppler image, the I and Q values of an ensemble are wall filtered prior to the estimation of the mean Doppler frequency or power. The wall filter has an adjustable cutoff frequency above or below which motion will be rejected such as the low frequency motion of the wall of a blood vessel when imaging flowing blood.

The contrast, B mode, and Doppler images are coupled to a display processor 40 which performs the processing needed to display the images on an image display 42. This may include displaying two images at the same time, side-by-side. It may also comprise overlaying perfusion parameter colors over the B mode images so that perfusion parameters are shown in relation to the tissue structure in which the contrast agent is located.

In accordance with the principles of the present invention, the ultrasound system of Fig. 1 also includes a Cineloop^{®} memory 44 and a PSF thinning processor 46. As real-time images are acquired by the system and displayed on the image display 42, they can simultaneously be stored as an image sequence (loop) in the Cineloop memory for later recall and review. For example, the live images of an entire CEUS exam can be saved in the Cineloop memory starting from prior to the arrival of contrast agent in the image field, and continuing through the wash-in and the completion of the wash-out phases of the perfusion cycle. The clinician can then replay the entire CEUS exam from memory at a later time or date such as when diagnosis is performed.

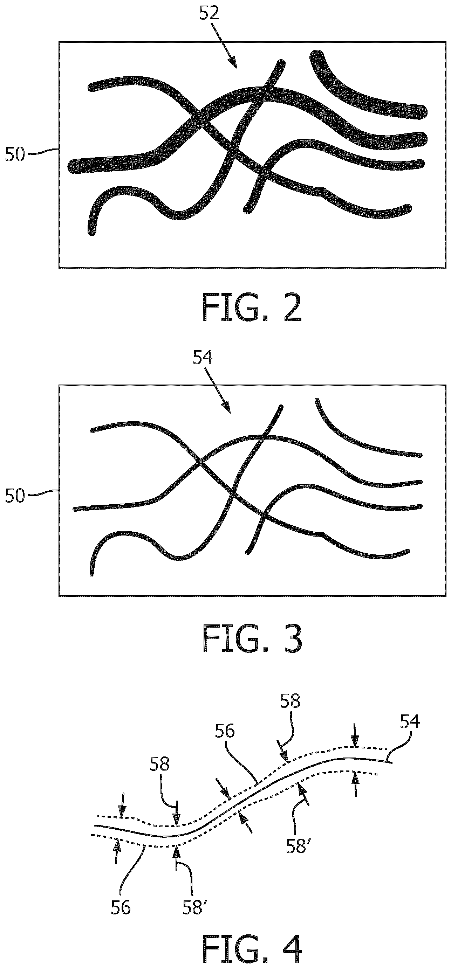

In accordance with a further aspect of the present invention, the images of a CEUS exam can be recalled from the Cineloop memory, processed by PSF thinning, and the microvessels shown in the images can be measured. In addition, before applying the PSF thinning processing, a maximum intensity projection technique, otherwise known as a "peak-hold" or "temporal accumulation" mode, can be applied to form a more complete representation of the underlying microvascular architecture. Microvessels of a selected range of size can then be used for parametric contrast assessment. In a preferred implementation of the present invention, the parameter measured is the diameter of the microvessels, although other parameters such as the radius, cross-sectional area, vessel width, or volume alternatively can be measured. The process for doing so is illustrated in the following drawings. Fig. 2 is a representation of an ultrasound image 50 of a group of microvessels 52 such as a group which is supplying blood to a lesion. As Fig. 2 illustrates, the microvessels can be of different sizes. The images of this group of microvessels can be recalled from the Cineloop memory in sequence and processed in the following manner to measure the diameters of the microvessels in the images. First, the microvessels of Fig. 2 are processed by the PSF thinning processor in what is known as skeletonization. In skeletonization, a blood vessel is reduced in size to only its central axis by PSF thinning. International patent pub. no. WO 2021/099372 A1 (Wang et al.) describes several different PSF thinning techniques, including the morphological operations of image erosion and image dilation, a spatial smoothing thinning technique, and a low pass filter thinning technique. The image erosion thinning technique may be used to erode away the outer circumference of the image of a microvessel, leaving only the center of the microvessel, a "skeleton" of the microvessel. Fig. 3 illustrates an ultrasound image 50 after the microvessels have been processed by the image erosion technique to leave only the central axes 54 of the vessels in the image 50. This skeletonized image is seen to be much more open than the tangle of microvessels of Fig. 2, and to better enable the individual microvessels to be distinguished in the image.

Fig. 4 illustrates an image of a microvessel which can now be measured to determine its diameter. The central axis 54 of a microvessel from Fig. 3, when combined with its outer periphery 56 from Fig. 2, enables the diameter of the vessel to be measured accurately, since the measurements are normal to and will pass through the center of the vessel delineated by the "skeleton." The orientation of the microvessel can be determined by finding the line of best fit to a localized series of skeleton points, then using this line to trace a perpendicular line to the vessel. The pairs of arrows 58, 58' along the length of the microvessel illustrate measurements of diameters of the microvessel at different points along the microvessel. The system may then select the mean of these measurements, the greatest of these measurements, or some other choice or combination of the measurements to represent the diameter of the microvessel.

Other techniques may also be employed to measure the size of a microvessel. For example, the ultrasound system of Fig. 1 can image the microvessels of Fig. 2 using three-dimensional color Doppler. When the blood flow is sufficient, the flow will be laminar and the highest velocity point in a cross-section of the microvessel will indicate the center of the image. Measurements can the be made of each microvessel which intersect a central high velocity point to determine the diameter of the microvessel.

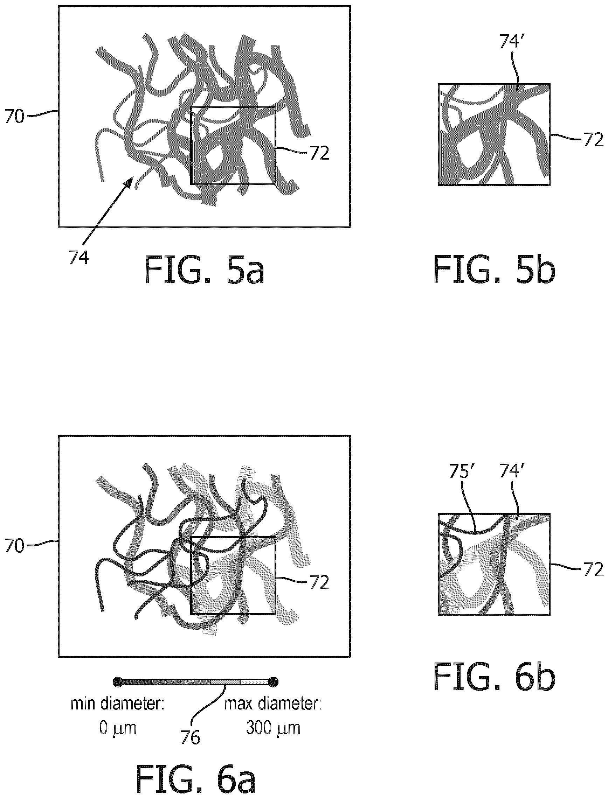

Once the diameters of the microvessels have been determined, an implementation of the present invention enables a group of microvessels to be classified by diameter. Fig. 5a illustrates an ultrasound image 70 of a group of microvessels 74. A clinician has placed a graphic box 72 over a portion of this image to delineate a specific group of microvessels for diagnosis. Fig. 5b is an enlarged view of the delineated region 72 of microvessels, including a relatively large vessel 74'.

Fig. 6a illustrates the images of Figs. 5a and 5b after the diameters of the microvessels have been measured. Now the microvessels are display in different colors, with the color-coding indicating their diameters over a range of microvessel diameters. The color of a microvessel corresponds to its size in the range of different shadings (sizes) of the color bar 76 below the image 70. At the left end of the color bar 76, microvessels are color-coded with a color starting from a minimum vessel diameter of 0 µm and continuing through a color indicating a maximum diameter of 300 µm. The enlarged view of the region of interest (ROI) box 72 shown in Fig. 6b contains the very large vessel 74' and a very small vessel 75' displayed in different colors. Color bar 76 and corresponding vessels have exemplary colors from left to right on the bar of black, tan, brown, magenta and red.

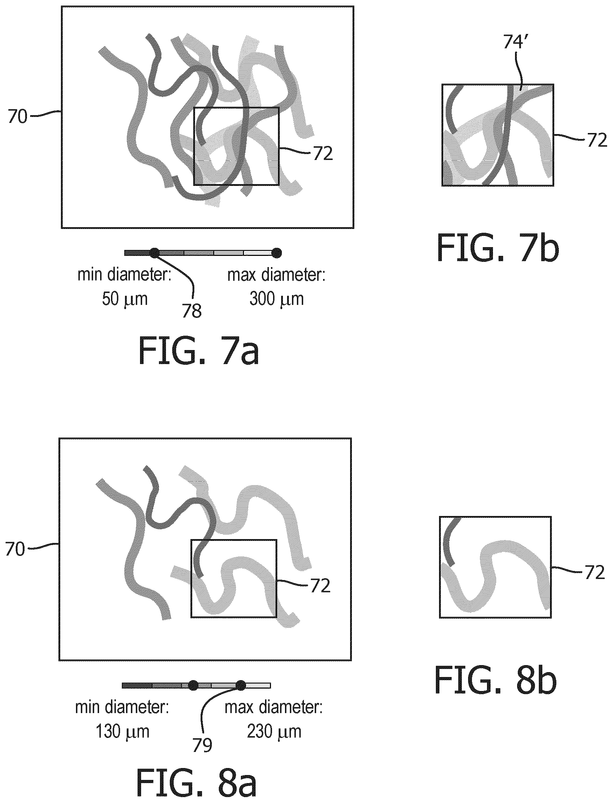

The large circles at each end of the color bar 76 of Fig. 6a are sliders which can be moved to more narrowly delineate the microvessel diametric range of interest. In Fig. 7a, the left slider 78 has been moved to the right to increase the minimum diameter of microvessels of interest. This change in the vessel diameters of interest has resulted in the elimination of microvessel 75' from the range of microvessel diameters of interest, as is seen by comparing Fig. 7b with Fig. 6b which illustrates the disappearance of microvessel 75' from the ROI.

In Fig. 8a the slider 79 has been moved to the left to reduce the maximum diameter of the microvessels of interest. Fig. 8b illustrates that the ROI 72 no longer shows the large vessel 74' and only two microvessels remain in the region of interest 72 of Fig. 8b. The result is that the succeeding parametric measures of blood flow will only include the blood flow of the microvessels shown in the ROI 72 of Fig. 8b.

Other possible setting configurations of the sliders 78,79 are readily apparent. For example a clinician concerned about the adverse effects of large vessels on parametric assessments of microvessels can just move the slider 79 to the left. This will eliminate the largest microvessels from a region of interest and thereby eliminate the effects of these large vessels on the succeeding measurements.

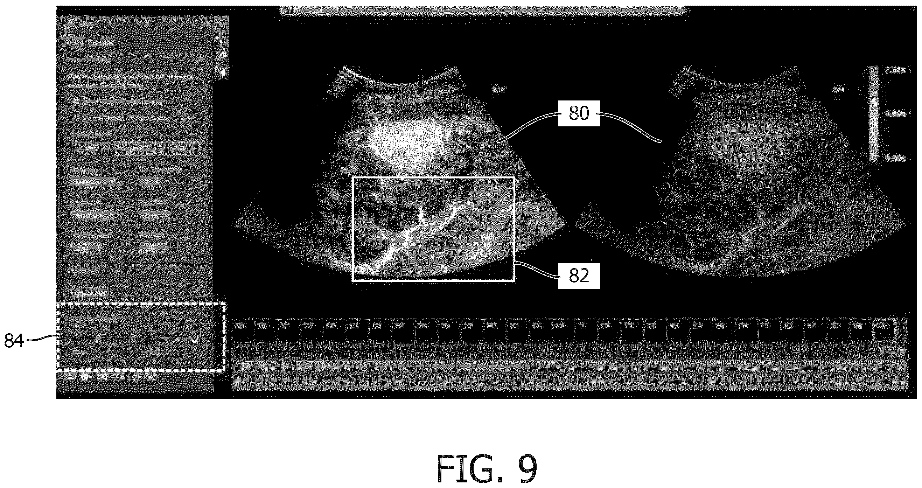

A more formal user interface of an ultrasound system constructed in accordance with the present invention is shown in Fig. 9. The illustrated user interface is capable of displaying two ultrasound images 80 side-by-side for comparative evaluation. In this example the image on the left is a maximum intensity projection image, and the image on the right is a parametric contrast image, a time-of-arrival parametric image of the CEUS data. The frame numbers of the Cineloop are displayed below the images. In this example the clinician has placed a ROI box 82 over the image on the left to select a group of microvessels for evaluation. The panel on the left side of the user interface includes a number of user controls. In the illustration the "Tasks" tab has been selected to present a number of task settings that are available to the clinician. One of the settings is "Enable Motion Compensation" which, when set, will align the anatomy of the Cineloop from frame to frame so that the clinician is consistently working with the same anatomical detail from frame to frame. The controls below the Display Mode settings include a control for selecting one of several PSF thinning algorithms.

Near the bottom of the left panel and outlined by a dashed box 84 are the sliders 78,79 for setting the desired range of microvessel diameters. The two rectangular sliders can be manipulated between a minimum diameter ("min") at the left side of the range of control, and a maximum diameter ("max") at the right side. The numerical values of min and max are determined by selecting the Controls tab of the panel, which illustrates adjustable numeric entry boxes for the two limit settings. Another approach is to click on the word min or max to pop up a display entry box where the numerical settings can be entered. Once the numerical limit values have been entered for the imaging session, a green checkmark is illuminated to the right of the sliders to indicate that this task has been done. The sliders can thereafter be manipulated to select a desired range of microvessel diameters within the minimum and maximum range limits. An alternative to a numerical approach is to allow the slider settings to be more subjective within a predetermined range between min and max, such as the entire range of microvessel measurements. Moving the sliders would then delineate a relative subset of the full range of values, without precise numerical indicators.

The user interface of Fig. 9 can be operated by a cursor or a finger on a touchscreen display, or by the trackball or a mouse on a standard ultrasound system display. The operation of the user interface, and of the measurement of a selected group of microvessels, is controlled by a microvessel measurement & selection processor 48 as shown in Fig. 1. When a clinician wants to perform a diagnosis in accordance with the present invention, a sequence of CEUS images of a microvasculature of interest is recalled from the Cineloop memory and replayed on the system display 42 using a user interface called up by processor 48, such as that shown in Fig. 9. Images 80 of Fig. 9 are typical Cineloop images of microvasculature. The image sequence from the Cineloop may be trimmed by the clinician to limit the loop to images of a perfusion cycle for diagnosis. The processor 48 enables the clinician to manipulate the ROI box 82 over the microvessels of interest and shown in Figs. 5b and 9, and the processor measures the sizes of the microvessels as described in Figs. 2-4. The measurement process may include skeletonizing the microvessels as described above, which is accomplished by the processor invoking operation of the PSF thinning processor, set to perform a desired thinning algorithm by the user interface. The processor 48 may color-code the vessels by size as shown in Figs. 6a and 6b, using colors of a color bar 76 consisting of distinct color hues. When the clinician manipulates the sliders 78,79 to select a desired range of microvessel sizes for the diagnosis, the processor 48 eliminates the out-of-range vessels from the ROI box 72 or 82, as shown in Figs. 7b and 8b.

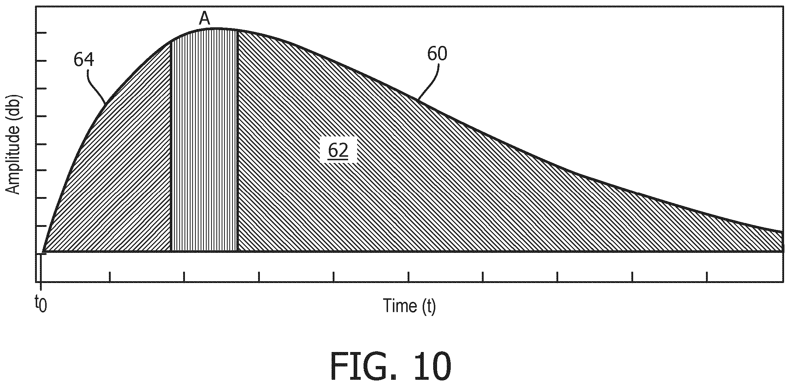

In accordance with a further aspect of the present invention, the ultrasound system of Fig. 1 includes a time-intensity curve (TIC) processor 49. The purpose of the TIC processor is to utilize nonlinear backscattered echoes of a sequence of CEUS images acquired during contrast agent wash-in and wash-out to produce a time-intensity curve of contrast agent perfusion such as that shown in Fig. 10. Time-intensity curves can be formed by TIC processor 49 for each point (pixel) in a contrast image, or by selected groups of pixels or the pixels of an entire image. Using the nonlinear backscattered signal amplitudes acquired during wash-in and wash-out of the contrast agent, curves of contrast intensity at each pixel location or group of pixel locations are calculated by the TIC processor as described in US pat. pub. no. 2011/0208061 (Chang). A time-intensity curve can be graphically displayed by graphics processor 26, which is coupled to the display processor 40 and the display 42. The time-intensity curve 60 of Fig. 10 is seen to start at time t₀ when contrast agent begins to arrive in ROI areas where the pixels selected for the curve are located. The perfusion curve 60 is seen to begin to build at starting time to and rise during contrast agent wash-in to a peak perfusion point A. Thereafter, as the passage of a bolus of contrast agent declines, the time-intensity curve declines steadily during the wash-out phase. Several metrics related to perfusion can be determined from the perfusion curve 60. One is the peak intensity A when the build-up of contrast agent perfusion is at a maximum. Another is the area under the perfusion curve, indicated by the cross-hatched areas 62. The left-most area is the area under the wash-in phase of contrast agent, the central area is the area under the peak perfusion phase, and the right-most area is the area under the wash-out phase of contrast flow. A third perfusion metric of clinical value is the slope of the perfusion curve 60 during contrast agent wash-in, which can be determined by measuring the slope of a line 64 which is tangential to the wash-in portion of curve 60.

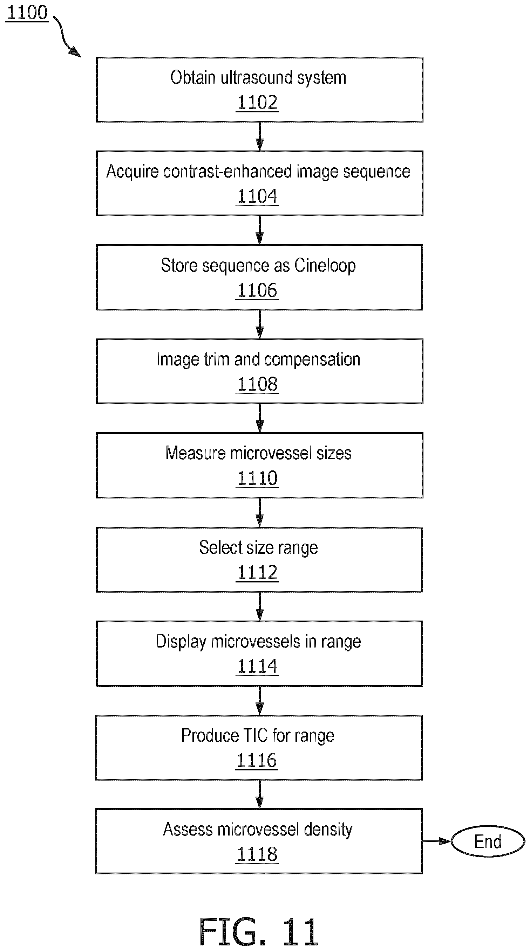

It is thus seen as illustrated in Fig. 11 that a clinician can use an ultrasound system of the present invention to perform the parametric evaluation of microvascular density outlined at the outset of this application. The procedure 1100 first comprises obtaining the afore-described ultrasound system at step 1102. The system then performs the step of acquiring 1104 a contrast-enhanced image sequence of a lesion and its microvasculature The sequence of images is then saved and stored in memory at 1106 as a loop of images in the Cineloop memory. The image loop is replayed from the memory and may optionally be trimmed and motion-compensated at 1108 as desired. The loop may also undergo maximum intensity projection processing at step 1108 to obtain a more complete image of the microvascular architecture. The microvessel measurement & selection processor then automatically measures the sizes of the microvessels in an image at 1110. An ROI box 82 may be located over the microvessels of interest in the image with the user interface. The clinician may then manipulate the sliders 78,79 at selecting step 1112 to select microvessels of a range of sizes for assessment. The loop of images acquired during contrast agent wash-in and wash-out is then replayed and may be displayed at 1114, and pixel intensities due to contrast perfusion in the selected vessels are used by the TIC processor to produce a time-intensity curve 60 at step 1116. The peak intensity A of the time-intensity curve and the area 62 under the time-intensity curve are used in the assessment 1118 of microvascular density in the region of interest.

It should be noted that an ultrasound system suitable for use in an implementation of the present invention, and in particular the component structure of the ultrasound system of Fig. 1, may be implemented in hardware, software, or a combination thereof. The various embodiments and/or components of an ultrasound system, for example, the transmit controller, the TIC processor, the contrast image processor, the graphics processor, the PSF thinning processor and the microvessel measurement & selection processor, and the components and controllers therein, also may be implemented as part of one or more computers or microprocessors. The computer or processor may include a computing device, an input device, a display unit and an interface, for example, for accessing the Internet. The computer or processor may include a microprocessor. The microprocessor may be connected to a communication bus, for example, to access a PACS system or a data network for importing training images and software. The computer or processor may also include a memory. The memory devices such as the Cineloop memory 44 may include Random Access Memory (RAM) and Read Only Memory (ROM). The computer or processor further may include a storage device, which may be a hard disk drive or a removable storage drive such as a floppy disk drive, optical disk drive, solid-state drive, and the like. The storage device may also be other similar means for loading computer programs or other instructions into the computer or processor.

As used herein, the term "computer" or "module" or "processor" or "workstation" may include any processor-based or microprocessor-based system including systems using microcontrollers, reduced instruction set computers (RISC), ASICs, logic circuits, and any other circuit or processor capable of executing the functions described herein. The above examples are exemplary only, and are thus not intended to limit in any way the definition and/or meaning of these terms.

The computer or processor executes a set of instructions that are stored in one or more storage elements, in order to process input data. The storage elements may also store data or other information as desired or needed. The storage element may be in the form of an information source or a physical memory element within a processing machine.

The set of instructions of an ultrasound system including those controlling the acquisition, processing, and display of ultrasound images as described above may include various commands that instruct a computer or processor as a processing machine to perform specific operations such as the methods and processes of the various embodiments of the invention. The set of instructions may be in the form of a software program. The software may be in various forms such as system software or application software and which may be embodied as a tangible and non-transitory computer readable medium. Further, the software may be in the form of a collection of separate programs or modules such as a neural network model module, a program module within a larger program or a portion of a program module. The software also may include modular programming in the form of object-oriented programming. The processing of input data by the processing machine may be in response to operator commands, or in response to results of previous processing, or in response to a request made by another processing machine.

Furthermore, the limitations of the following claims are not written in means-plus-function format and are not intended to be interpreted based on 35 U.S.C. 112, sixth paragraph, unless and until such claim limitations expressly use the phrase "means for" followed by a statement of function devoid of further structure.

## Claims

1. An ultrasonic diagnostic imaging system for conducting contrast-enhanced ultrasound imaging and assessment of microvasculature comprising:
an ultrasound probe (10) adapted to acquire ultrasonic echo signals from an image field experiencing a flow of a microbubble contrast agent;
a beamformer (30) coupled to the ultrasound probe and adapted to produce beamformed echo signals;
a contrast image processor (38) coupled to the beamformer, and adapted to produce images of microvessels perfused with the microbubble contrast agent;
an image display (42) coupled to the contrast image processor and adapted to display the images of the microvessels perfused with the microbubble contrast agent;
a memory (44), coupled to the contrast image processor, and adapted to store a sequence of the images of anatomy perfused with the microbubble contrast agent acquired during wash-in of the microbubble contrast agent; and
a microvessel measurement & selection processor (48), coupled to the memory, and adapted to enable selection of a group of microvessels for measurement and to measure sizes of the microvessels, wherein the microvessel measurement & selection processor is further adapted to enable selection of a range of microvessel sizes for assessment.

2. The ultrasonic diagnostic imaging system of Claim 1, further comprising a PSF thinning processor adapted to perform PSF thinning of images of microvessels, and optionally, wherein the PSF thinning processor is further adapted to skeletonize the images of a selected group of microvessels.

3. The ultrasonic diagnostic imaging system of Claim 1, wherein the microvessel measurement & selection processor is further adapted to color images of a selected group of microvessels in accordance with sizes of the microvessels; and optionally to prevent the display of microvessels of the selected group of microvessels which are not within a selected range of microvessel sizes.

4. The ultrasonic diagnostic imaging system of Claim 1, further comprising a user interface, operable in conjunction with the microvessel measurement & selection processor, and adapted for operation by a user to delineate a range of microvessel sizes for assessment.

5. The ultrasonic diagnostic imaging system of Claim 4, wherein the user interface is further operable by a user to position two sliders in positions which delineate a maximum microvessel size and a minimum microvessel size, respectively.

6. The ultrasonic diagnostic imaging system of Claim 5, wherein the user interface is further operable to display images of the sequence of images acquired during wash-in of the contrast agent, and optionally to display a replay of images of the sequence of images acquired during wash-in of the contrast agent and stored in the memory.

7. The ultrasonic diagnostic imaging system of Claim 6, wherein the user interface is further operable to enable a user to designate a region of an image replayed from the memory as a region of interest for microvessel assessment.

8. The ultrasonic diagnostic imaging system of Claim 4, wherein the user interface is further adapted to display both a maximum intensity projection image and a parametric contrast image.

9. The ultrasonic diagnostic imaging system of Claim 1, further comprising a time-intensity curve processor, responsive to echo signals returned from the microbubble contrast agent, and adapted to produce a time-intensity curve of microbubble contrast agent wash-in and wash-out.

10. The ultrasonic diagnostic imaging system of Claim 9, wherein the time-intensity curve processor is further responsive to image intensity of a sequence of images, acquired during contrast agent wash-in and stored in the memory, and replayed from the memory, for the production of a time-intensity curve.

11. The ultrasonic diagnostic imaging system of Claim 10, wherein the time-intensity curve processor is further responsive to a time-intensity curve produced by the processor for measuring a peak of the curve and an area under the curve.

12. The ultrasonic diagnostic imaging system of Claim 11, further comprising a graphics processor adapted to process a time-intensity curve produced by the time-intensity curve processor for display.

13. The ultrasonic diagnostic imaging system of Claim 1, further comprising a Doppler processor adapted for producing colorflow images of the microvessels perfused with the microbubble contrast agent.

14. A method (1100) for conducting contrast-enhanced ultrasound imaging and assessment of microvasculature comprising:
acquiring (1104) with an ultrasound system a sequence of images of microvessels perfused with a micro-bubble contrast agent;
storing (1106) the sequence of images in a memory;
automatically measuring (1110) a size of each of the microvessels;
selecting (1112) in response to a user interface input a range of sizes of the microvessels for display; and
displaying (1114) only the microvessels with a size within the range of sizes in at least one of the sequence of images.

15. The method of Claim 14, further comprising the steps of;
producing (1116) a time-intensity curve from the selected microvessels; and
assessing (1118) a microvascular density using the time-intensity curve ; and/or
locating a region-of-interest (ROI) box over the image, and wherein the measuring step includes only the microvessels in the ROI.
